Europäisches Patentamt

European Patent Office

Office européen des brevets

(11) Veröffentlichungsnummer : **0 029 166 B1**

# EUROPÄISCHE PATENTSCHRIFT

(45) Veröffentlichungstag der Patentschrift :
20.04.83

(51) Int. Cl.³ : **A 61 B 5/02, A 61 B 7/00**

(21) Anmeldenummer : **80106785.1**

(22) Anmeldetag : **04.11.80**

(54) **Elektronisches Blutdruckmessgerät.**

(30) Priorität : 08.11.79 DE 2945126

(43) Veröffentlichungstag der Anmeldung :
27.05.81 Patentblatt 81/21

(45) Bekanntmachung des Hinweises auf die Patenterteilung : 20.04.83 Patentblatt 83/16

(84) Benannte Vertragsstaaten :
**AT BE CH DE FR GB IT LI LU NL SE**

(56) Entgegenhaltungen :
**DE A 2 041 916**
**DE A 2 822 194**
**FR A 2 430 756**
**GB A 1 481 117**
**US A 3 308 811**
**US A 3 763 851**
**US A 3 934 577**

**MEDICAL AND BIOLOGICAL ENGINEERING,**
**Band 12, Nr. 4, Juli 1974, Seiten 479-482 Steven-**
**age, G.B. A.J. COUSIN et al. : « A cardiac prejec-**
**tion period monitor for foetal assessment during**
**labour »**

(73) Patentinhaber : **BOSCH + SOHN Fabrik mediz. Appa-**
**rate GmbH & Co., KG**
**Postfach 34 Bahnhofstrasse 64**
**D-7455 Jungingen (DE)**

(72) Erfinder : **Weltersbach, Helmer, Dipl.-Ing.**
**De Lange Akker 158**
**Vaals (NL)**

(74) Vertreter : **Patentanwälte TER MEER - MÜLLER -**
**STEINMEISTER**
**Triftstrasse 4**
**D-8000 München 22 (DE)**

Elektronisches Blutdruckmessgerät

Die Erfindung betrifft ein elektronisches Blutdruckmeßgerät mit aufblasbarer Manschette und einem Meßwertgeber, der Korotkoff-Signale und Herzfrequenzsignale erfaßt, die über eine Filteranordnung mit nachgeschalteter Signalverarbeitung selektiv aufbereitet und einer Meßwertanzeigevorrichtung zugeführt werden.

Typische Beispiele für diese Art von elektronischen Blutdruckmeßgeräten sind in der DE-OS 25 15 868 und der DE-OS 20 41 916 beschrieben : Die von einem Meßwertgeber beispielsweise von einem Mikrophon, das an einer von der Manschette herzab liegenden Stelle angesetzt wird, gelieferten Signale werden zwei Bandpaßfiltern angeboten, von denen das eine auf die Grundfrequenz der Pulsschlaggeräusche, das heißt auf etwa 5 bis 10 Hz abgestimmt ist, während das andere Bandpaßfilter eine Mittenfrequenz von etwa 40 bis 200 Hz aufweist und damit die Grundwelle der Korotkoff-Signale erfaßt. Über eine nachgeschaltete digitale Schaltkreislogik lassen sich dann bei ununterbrochenem sukzessivem Auftreten der ersten zwei oder drei Korotkoff-Signale einerseits der systolische Druckwert und andererseits beim Verschwinden der Korotkoff-Signale während zweier oder dreier Herzschlagsignale der diastolische Druckwert erfassen und auf einem Registriergerät anzeigen.

Bekannte elektronische Blutdruckmeßgeräte, die nach diesem Prinzip mit zwei fest eingestellten Bandpaßfiltern arbeiten, liefern zufriedenstellende Ergebnisse bei der sogenannten Ruhemessung, das heißt, wenn sich die Person, deren Blutdruck gemessen werden soll, mehr oder weniger im Ruhezustand befindet, also unmittelbar vor oder während der Messung keiner besonderen körperlichen Anstrengung ausgesetzt ist. Sehr häufig ist es jedoch erwünscht, den Blutdruck unmittelbar im Anschluß oder während eines körperlichen Belastungszustands zu überwachen. In diesem Fall werden die Pulsgeräusche aufgrund des erhöhten Herzschlagvolumens wesentlich stärker und die Frequenzbereiche vor allem von Puls und Diastole können sich mehr oder weniger stark überlappen, weil sich die maximale Herzfrequenz nicht mehr deutlich von der niedrigsten erfaßten Grundfrequenz des diastolischen Druckwerts unterscheidet. In anderen Worten : Bei den bisher verwendeten Filtern können die Pulsgeräusche selbst bei amplitudenabhängiger Aussteuerung nach dem Erreichen der Diastole in den Übertragungsbereich beider Filter fallen, weil diese während des ganzen Meßvorgangs den fest vorgegebenen Frequenzbereich übertragen. Vor allem bei sogenannten Ergometriemessungen ist dann eine eindeutige Zuordnung von Systole und Diastole nicht mehr möglich.

Der Erfindung liegt damit die Aufgabe zugrunde, ein elektronisches, vorzugsweise vollautomatisch arbeitendes Blutdruckmeßgerät zu schaffen, bei dem eine einwandfreie Trennung von Systole und Diastole auch bei einer Blutdruckmessung kurz nach oder während einer stärkeren körperlichen Belastung (Ergometrie) möglich ist. Insbesondere soll bei der Blutdruckmessung während der Ergometrie verhindert werden, daß Pulsgeräusche fälschlicherweise als diastolischer Druckwert interpretiert werden bzw. die Erkennung der Diastole verhindern. Das Gerät soll sowohl bei Ruhemessung als auch bei Belastungsmessung eine einwandfreie Unterscheidung und Anzeige der beiden für eine Blutdruckmessung charakteristischen Druckwerte gewährleisten.

Die erfindungsgemäße Lösung der gestellten Aufgabe ist in kurzer Zusammenfassung mit ihren erfindungswesentlichen Merkmalen im Anspruch 1 angegeben.

Vorteilhafte Weiterbildungen des Erfindungsgedankens sind in Unteransprüchen gekennzeichnet.

Um einer Lösung der gestellten Aufgabe näherzukommen, wurden zunächst Voruntersuchungen durchgeführt, die zu dem Ergebnis führten, daß für den systolischen Druckwert andere Grundfrequenzen maßgeblich sind als für den diastolischen Druckwert. Die beiden Frequenzwerte liegen, wie durch Reihenmessungen festgestellt wurde, im Bereich von 10 bis 100 Hz, das heißt, der gesamte maßgebliche Frequenzbereich ist enger als bisher vermutet, wobei für den systolischen Druckwert individuell unterschiedliche Frequenzen von 10 bis ca. 40 Hz maßgeblich sind, während für den diastolischen Druckwert Grundfrequenzen von 60 bis 80 Hz, in Ausnahmefällen bis ca. 100 Hz zu beobachten sind, die wieder abhängig von der jeweiligen Pulsfrequenz des Patienten sind.

Durch die erfindungsgemäße Lösung, nämlich eine Schaltungsanordnung, mit der sich die Mittenfrequenz eines Bandpasses höherer Ordnung (z. B. n ≥ 8) in Abhängigkeit von der Pulsfrequenz sukzessiv während der Druckmessung verändern läßt, wobei sich die Abhängigkeit vom Druckwert ebenfalls berücksichtigen läßt, kann eine signifikante Zuordnung von systolischem und diastolischem Druckwert auch bei starker körperlicher Belastung gewährleistet werden, wie sie beispielsweise während der Ergometrie auftritt.

Ein zusätzlicher Vorteil der Erfindung ergibt sich aufgrund der pulsfrequenzabhängigen Verschiebung der Filtermittenfrequenz durch eine Reduzierung von Artefaktstörungen, die im gesamten Nutzspektrum vorkommen können und bei bekannten elektronischen Blutdruckmeßgeräten ebenfalls Ursache für Fehlinterpretationen, namentlich hinsichtlich des diastolischen Druckwerts waren.

Bei einer vorteilhaften Ausgestaltungsform der Erfindung besteht die Filteranordnung aus einem digital steuerbaren Filter, bei dem durch Änderung der Abtastfrequenz (z. B. von 2 kHz bis 200 kHz) sich ein Bandpaß mit dem Mittenfrequenzbereich von 20 bis 200 Hz hoher Güte realisieren läßt.

Eine andere vorteilhafte Ausführungsform der Erfindung sieht die Verwendung eines aktiven Filters vor, das im Prinzip ein Filter n-ter Ordnung z. B. mit $n \geq 8$ sein kann und bei dem passive Schaltkreiselemente über die Steuereinrichtung selektiv zuschaltbar sind.

Die Erfindung und vorteilhafte Einzelheiten werden nachfolgend unter Bezug auf die Zeichnung in beispielsweisen Ausführungsformen näher erläutert. Es zeigen :

Figur 1 die wesentlichen Baugruppen einer ersten Ausführungsform eines erfindungsgemäßen elektronischen Blutdruckmeßgerätes mit diskreten, in Abhängigkeit von der Pulsfrequenz selektiv wirksam schaltbaren passiven Elementen ;

Figur 2 eine andere Ausführungsform der Erfindung mit einer sogenannten SCF-, CCD- oder CTD-Filter-Anordnung, die in drei Gruppen unterteilt ist und zum einen ein umschaltbares Bandpaßfilter geringer Ordnung (Antialiasing-Filter), das eigentliche Bandpaßfilter sowie einen umschaltbaren Tiefpaß zur Unterdrückung der multiplikativen Anteile der Abtastfrequenz umfaßt ; und

Figur 3 ein schaltungstechnisches Ausführungsbeispiel für wesentliche Baugruppen bei den Ausführungsformen nach Fig. 1 bzw. Fig. 2.

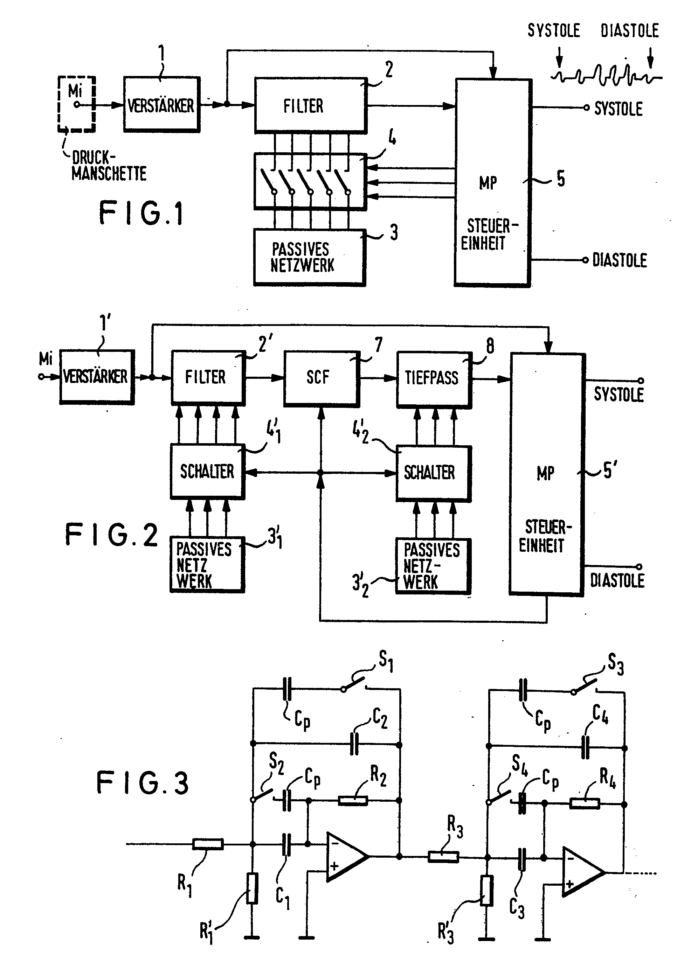

Zunächst wird die Ausführungsform der Erfindung nach Fig. 1 näher betrachtet :

Die durch ein Mikrophon Mi herzab von oder in der Druckmanschette aufgenommenen Signale gelangen auf einen Mikrophonverstärker 1, der so ausgelegt ist, daß eine Übersteuerung des nachfolgenden Filters 2 nicht möglich ist, zum Beispiel durch Verwendung eines Operationsverstärkers mit Bias-Eingang. Das vom Mikrophonverstärker 1 gespeiste Filter 2 ist ein Bandpaß höherer Ordnung (z. B. n = 8), bei dem in Abhängigkeit von der Pulsfrequenz, die durch eine weiter unten noch näher erläuterte Steuereinheit 5 abgetastet wird, die Mittenfrequenz während eines Meßvorgangs stetig oder abschnittweise verändert wird. Diese Änderung der Mittenfrequenz während der Messung erfolgt beim Ausführungsbeispiel der Fig. 1 dadurch, daß mit Hilfe eines zusätzlichen R- und/oder C-Netzwerks in Verbindung mit elektronischen Schaltern 4 die Übertragungsfunktion geändert wird.

Durch diese laufende Anpassung der Übertragungsfunktion und damit der Mittenfrequenz während der Messung ist eine signifikante Zuordnung von Systole und Diastole insbesondere auch bei starker körperlicher Belastung möglich, wie sie etwa während der Ergometrie auftritt. Die Frequenzbereiche von Puls und Diastole können sich jetzt nicht mehr überlappen, weil sich die maximale Herzfrequenz deutlich von der niedrigsten erfaßten Grundfrequenz des diastolischen Druckwerts unterscheiden läßt.

Die Steuereinheit 5 ist bei einer vorteilhaften Ausgestaltungsform der Erfindung als integrierte Schaltung ausgelegt und beispielsweise Teil eines Mikroprozessor-Chips. Dieser Steuereinheit werden als Eingangsgrößen einmal die ungefilterten Ausgangssignale des Mikrophonverstärkers 1 und zum anderen die Ausgangssignale des Filters 2 zugeführt. Die Steuereinheit 5 liefert als Ergebnis eines Rechen- und Umsetzungsvorgangs selektiv wirkende Steuersignale an die elektronischen Schalter 4, über welche Teile des Netzwerks 3 auswahlweise mit dem Filter 2 verbindbar sind und dadurch dessen Übertragungsfunktion ändern. Am Ausgang der Steuereinheit 5 erscheinen dann vorzugsweise auf getrennten Ausgangsanschlüssen die Signale für die Systole bzw. die Diastole, und zwar entweder als Digitalwerte oder bereits als über D/A-Wandler umgesetzte Analogwerte, die unmittelbar entsprechend auf ein digitales bzw. analoges Anzeigegerät schaltbar sind.

Die Blockschaltbilddarstellung der Fig. 2 zeigt ein anderes Ausführungsbeispiel, welches sich gegenüber der Ausführungsform nach Fig. 1 durch bessere Selektion auszeichnet, sich aber, da in integrierter Technik herstellbar, auch noch billiger realisieren läßt. Um die prinzipielle Verwandtschaft mit der Lösung nach Fig. 1 zu verdeutlichen, sind die in ihrer Funktion entsprechenden, in der Regel aber anders dimensionierten Baugruppen, die aus der Schaltung der Fig. 1 bereits bekannt sind, mit der gleichen, jedoch um einen Strich (') ergänzten Markierung versehen.

Das wesentliche Unterscheidungsmerkmal zur Lösung nach Fig. 1 ist hier die Verwendung eines Filters mit einem Switched-Capacitor-Filterbaustein. Dieses Filter umfaßt den vom Mikrophon Mi gespeisten, nicht übersteuerbaren Mikrophonverstärker 1', ein schaltbares Antialiasing-Filter 2', also ein umschaltbares Bandpaß-Filter geringer Ordnung, ein Switched-Capacitor-Filter 7 (nachfolgend « SCF ») und einen umschaltbaren Tiefpaß 8. Die Mittenfrequenz des SCF 7, welches als Bandpaß sehr hoher Ordnung wirkt, wird von der Steuereinheit 5' aus während der Messung geändert. Entsprechend muß auch die Übertragungsfunktion des Antialiasing-Filters 2' bzw. des Tiefpasses 8 geändert werden, welcher zur Unterdrückung der multiplikativen Anteile der Abtastfrequenz dient und als Tiefpaß hoher Ordnung ausgelegt ist. Während die Mittenfrequenz des SCF 7 durch ein veränderliches Trigger-Signal von der Steuereinheit 5' in Abhängigkeit von der Pulsfrequenz aus eingestellt wird, erfolgt das Nachziehen der Übertragungsfunktionen des Antialiasing-Filters 2' bzw. des Tiefpaßfilters 8 über ein jeweils zugeordnetes selektiv vom Triggersignal umschaltbares R- und/oder C-Netzwerk $3'_1$ bzw. $3'_2$, ähnlich wie bei der Ausführungsform nach Fig. 1, mittels elektronischer Schalter $4'_1$ bzw. $4'_2$. Dieses Filter stellt sicher, daß als Eingangssignale auftretende Frequenzanteile, die die Hälfte der Abtastfrequenz überschreiten, nicht als Summen- oder Differenzsignal in den Nutzbereich fallen. Gegenüber der Lösung nach Fig. 1 ergibt sich der Vorteil einer noch höheren Selektivität (Trennschärfe). Die gesamte Schaltung läßt sich in integrierter Technik und preiswert herstellen. Das SCF 7 kann auch durch ein CCD-Filter (CCD = Charge-Coupled Device) oder eine CTD-Filteranordnung (CTD = Charge-Transfer-Device) ersetzt sein.

Zur Funktionsweise sei ergänzend betont, daß die Übertragungsfunktionen des Antialiasing-Filterteils 2' und des Tiefpaßfilterteils 8 in Abhängigkeit von der Mittenfrequenz des SCF 7 durch das Netzwerk 3'$_1$ bzw. 3'$_2$ über die elektronischen Schalter 4'$_1$ bzw. 4'$_2$ eingestellt werden. Die Mittenfrequenz des SCF 7 ist — wie erwähnt — durch ein veränderliches Triggersignal bestimmt, das in Abhängigkeit von der Pulsfrequenz erzeugt wird, die unmittelbar am Ausgang des Mikrophonverstärkers 1' ableitbar ist. Zur Steuerung des Triggersignals kann ggfs. auch der Blutdruckwert ergänzend berücksichtigt werden.

Die Fig. 3 zeigt ein Ausführungsbeispiel für die Baugruppen 2, 3 und 4 in Fig. 1 bzw. für die Baugruppen 2', 4'$_1$, 3'$_1$ und 8, 4'$_2$, 3'$_2$ in Fig. 2.

Für den Fachmann ist ersichtlich, daß es sich bei dieser Schaltung um ein aktives Filter handelt, das im Prinzip ein Filter n-ter Ordnung mit bekannten Übertragungsfunktionen sein kann (n ganzzahlig). Die in dem Schaltbild enthaltenen Schalterelemente S$_1$, S$_2$, ... sind Halbleiterschalter der Baugruppe 4 in Fig. 1 bzw. 4'$_1$ und 4'$_2$ nach Fig. 2. Die als Zuschaltkapazitäten C$_P$ (Reihen oder Parallelkapazitäten) dargestellten Schaltelemente sind in dem passiven Netzwerk 3 in Fig. 1 bzw. 3'$_1$ und 3'$_2$ in Fig. 2 enthalten. Eine vorteilhafte Lösung kann sich auch dann ergeben, wenn statt der oder in Verbindung mit den zuschaltbaren Kapazitäten C$_P$ Widerstände zugeschaltet werden. Dies hat gegebenenfalls den Vorteil einer höheren Genauigkeit, da sich bei vergleichbarem Aufwand Widerstandsnetzwerke in der Regel präziser verwirklichen lassen als Kondensatorelemente. Die Widerstandselemente R$_1$, R$_2$ ... bzw. die Kondensatorelemente C$_1$, C$_2$ ... sind diskrete Bauelemente.

Wie dem Fachmann bekannt ist, lassen sich mit derartigen aktiven Filtern Tschebycheff-, Bessel-, Butterworth- und elliptische — im Prinzip also beliebige Filterfunktionen höherer Ordnung verwirklichen.

## Ansprüche

1. Elektronisches Blutdruckmeßgerät mit aufblasbarer Manschette und einem Meßwertgeber, der Korotkoff-Signale und Herzfrequenz-Signale erfaßt, die über eine Filteranordnung mit nachgeschalteter Signalverarbeitung selektiv aufbereitet und einer Meßwertanzeigevorrichtung zugeführt werden, dadurch gekennzeichnet, daß die Filteranordnung (2; 2', 7, 8) als Bandpaß höherer Ordnung ausgelegt ist, dessen Mittenfrequenz in Abhängigkeit von der Pulsfrequenz und/oder des Drucks sukzessiv nachführbar ist.

2. Blutdruckmeßgerät nach Anspruch 1, dadurch gekennzeichnet, daß die Mittenfrequenz durch eine die Pulsfrequenz erfassende Steuereinrichtung (5; 5') nachführbar ist.

3. Blutdruckmeßgerät nach Anspruch 1, dadurch gekennzeichnet, daß die Mittenfrequenz durch eine den Druckwert erfassende Steuereinrichtung nachführbar ist.

4. Blutdruckmeßgerät nach einem der vorstehenden Ansprüche, dadurch gekennzeichnet, daß der Durchlaßbereich des Bandpaßfilters unter Beibehaltung der Filterparameter änderbar ist.

5. Blutdruckmeßgerät nach einem der vorstehenden Ansprüche, dadurch gekennzeichnet, daß die Filteranordnung ein aktives Filter n-ter Ordnung mit n ⩾ 8 enthält, bei dem mindestens einzelne der passiven Bauelemente von der Steuereinheit (5; 5') aus über Schalter (4; 4'$_1$, 4'$_2$; S$_1$, S$_2$, ...) selektiv zuschaltbar sind.

6. Blutdruckmeßgerät nach Anspruch 5, dadurch gekennzeichnet, daß die Schalter als elektronische Schalterelemente ausgebildet sind.

7. Blutdruckmeßgerät nach einem der vorstehenden Ansprüche, dadurch gekennzeichnet, daß die Filteranordnung einen Bandpaß geringer Ordnung (2') mit durch selektiv zuschaltbaren passiven Schaltkreiselementen veränderbarer Mittenfrequenz, einen nachgeschalteten Bandpaß hoher Ordnung (7) mit durch ein Triggersignal einstellbarer Mittenfrequenz sowie ein diesem nachgeschaltetes Tiefpaßfilter (8) umfaßt, dessen Grenzfrequenz durch selektiv zu- oder abschaltbare passive Schaltkreiselemente veränderbar ist.

8. Blutdruckmeßgerät nach Anspruch 7, dadurch gekennzeichnet, daß der Bandpaß (2') geringer Ordnung ein Antialiasing-Filter und der nachgeschaltete Bandpaß (7) hoher Ordnung ein Switched-Capacitor-Filter oder eine CTD-Charge-Transfer-Device oder ein CCD-Charge-Coupled-Device-Filter ist.

9. Blutdruckmeßgerät nach einem der vorstehenden Ansprüche, dadurch gekennzeichnet, daß die in der Filteranordnung selektiv zu- oder abschaltbaren passiven Bauelemente als Kondensator-(Cp) und/oder als Widerstandselemente ausgebildet sind.

10. Blutdruckmeßgerät nach einem der vorstehenden Ansprüche, dadurch gekennzeichnet, daß mindestens Teile der Filteranordnung und/oder der Steuereinheit als integrierte Schaltung ausgeführt sind.

## Claims

1. Electronic blood pressure measuring instrument with inflatable cuff and a transducer which picks up Korotkoff signals and heart rate signals which via a filter arrangement with series-connected signal processing are selectively processed and fed to a measured value display device, characterised in that the filter arrangement (2; 2', 7, 8) is designed as a higher-level bandpass filter the mean frequency of which can be successively adjusted in dependence on the pulse rate and/or the pressure.

2. Blood pressure measuring instrument according to claim 1, characterised in that the mean frequency can be adjusted by means of a control device (5 ; 5') which picks up the pulse rate.

3. Blood pressure measuring instrument according to claim 1, characterised in that the mean frequency can be adjusted by a control device which picks up the pressure value.

4. Blood pressure measuring instrument according to one of the preceding claims, characterised in that the bandpass filter pass region is variable while the filter parameters remain unchanged.

5. Blood pressure measuring instrument according to one of the preceding claims, characterised in that the filter arrangement contains an active n-level filter where $n \geqslant 8$, in which at least some of the passive components can be selectively connected by the control unit (5 ; 5') via switches (4 ; $4'_1$, $4'_2$ ; $S_1$ ; $S_2$, ...).

6. Blood pressure measuring instrument according to claim 5, characterised in that the switches are in the form of electronic switching elements.

7. Blood pressure measuring instrument according to one of the preceding claims, characterised in that the filter arrangement includes a low-level bandpass filter (2') having a mean frequency which can be varied by means of selectively connectable passive circuit elements, a high-level band pass filter (7), connected in series, and having a mean frequency adjustable by a trigger signal, and a low-pass filter (8) whose cut-off frequency is variable by passive circuit elements which can be selectively switched on or off.

8. Blood pressure measuring instrument according to claim 7, characterised in that the low-level bandpass filter (2') is an anti-aliasing filter and the high-level bandpass filter (7) connected in series is a switched capacitor filter or a CTD (charge transfer device) or a CCD (charge coupled device) filter.

9. Blood pressure measuring instrument according to one of the preceding claims, characterised in that the passive components of the filter arrangement which can be selectively switched on or off are constructed as capacitor (Cp) and/or resistance elements.

10. Blood pressure measuring instrument according to one of the preceding claims, characterised in that at least parts of the filter arrangement and/or of the control unit are designed as an integrated circuit.

## Revendications

1. Appareil électronique de mesure de pression sanguine, comportant une garniture gonflable et un capteur de mesure qui détecte les signaux Korotkoff et les signaux de fréquence cardiaque, qui sont traités sélectivement dans un dispositif de filtrage suivi d'un traitement de signal, et amenés à un dispositif d'affichage de mesure, caractérisé par le fait que l'agencement de filtrage (2 ; 2', 7, 8) est réalisé sous la forme de passe-bande d'ordre élevé, dont la fréquence moyenne est susceptible d'être modifiée successivement en fonction de la fréquence du pouls et/ou de la pression.

2. Appareil de mesure de pression sanguine selon la revendication 1, caractérisé par le fait que la fréquence moyenne est susceptible d'être modifiée par un dispositif de commande (5 ; 5') détectant la fréquence du pouls.

3. Appareil de mesure de la pression sanguine selon la revendication 1, caractérisé par le fait que la fréquence moyenne est susceptible d'être modifiée par un dispositif de commande détectant la valeur de la pression.

4. Appareil de mesure de pression sanguine selon l'une des revendications précédentes, caractérisé par le fait que la bande passante du filtre passe-bande est susceptible d'être modifiée en conservant les paramètres du filtre.

5. Appareil de mesure de pression sanguine selon l'une des revendications précédentes, caractérisé par le fait que l'agencement de filtrage comporte un filtre actif d'ordre n avec $n \geqslant 8$, dans lequel au moins certains des composants passifs sont susceptibles d'être mis en circuit sélectivement par le dispositif de commande (5 ; 5') au travers de commutateurs (4 ; $4'_1$, $4'_2$ ; $S_1$, $S_2$, ...).

6. Appareil de mesure de pression sanguine selon la revendication 5, caractérisé par le fait que les commutateurs sont réalisés sous forme d'éléments de commutation électroniques.

7. Appareil de mesure de pression sanguine selon l'une des revendications précédentes, caractérisé par le fait que l'agencement de filtrage comprend un passe-bande d'ordre faible (2') avec une fréquence moyenne susceptible d'être modifiée par des éléments de circuit passifs pouvant être mis en circuit sélectivement, un passe-bande d'ordre élevé (7), à la suite, avec une fréquence moyenne susceptible d'être établie par un signal de déclenchement, et un filtre passe-bas (8), à la suite, dont la fréquence limite est susceptible d'être modifiée par des éléments de circuit passifs pouvant être mis sélectivement en ou hors circuit.

8. Appareil de mesure de pression sanguine selon la revendication 7, caractérisé par le fait que le passe-bande (2') d'ordre faible est un filtre Antialiasing et le passe-bande (7) d'ordre élevé, à la suite, est un filtre Switched-Capacitor ou un filtre CTD-Charge-Transfer-Device ou un filtre CCD-Charge-Coupled-Device.

9. Appareil de mesure de pression sanguine selon l'une des revendications précédentes, caractérisé par le fait que les éléments passifs susceptibles d'être mis en ou hors circuit sélectivement dans l'agencement de filtrage sont réalisés sous la forme de condensateurs (Cp) et/ou de résistances.

10. Appareil de mesure de pression sanguine selon l'une des revendications précédentes, caractérisé par le fait qu'au moins des parties de l'agencement de filtrage et/ou du dispositif de commande sont réalisées sous la forme de circuits intégrés.

FIG.1

FIG.2

FIG.3